# EUROPEAN PATENT SPECIFICATION

(11) **EP 1 243 923 B1**
(45) Date of publication and mention of the grant of the patent: **08.03.2006**
(21) Application number: 00981763.6
(22) Date of filing: 15.12.2000
(51) Int. Cl.: G01N 33/50, G01N 33/15, G01N 33/566, C12Q 1/02, C12N 15/09, C12N 5/10

(54) **METHOD OF SCREENING REMEDY FOR CANCER WITH THE USE OF INTERACTION DOMAINS OF p53 AND MORTALIN**
VERFAHREN ZUM AUFFINDEN EINES HEILMITTELS FÜR KREBS MIT HILFE VON INTERAKTIONSDOMÄNEN VON p53 UND MORTALIN
METHODE DE SELECTION D'UN REMEDE CONTRE LE CANCER UTILISANT DES DOMAINES D'INTERACTION DE P53 ET DE LA MORTALINE

(30) Priority: 16.12.1999 JP 35754599
(43) Date of publication of application: 25.09.2002
(73) Proprietor: CHUGAI SEIYAKU KABUSHIKI KAISHA, Tokyo, 115-8543 (JP)
(72) Inventor: WADHWA, Renu, Chugai Seiyaku Kabushiki Kaisha, Niihari-gun, Ibaraki 300-4101 (JP)
(74) Representative: Vossius & Partner
(86) International application number: PCT/JP2000/008910
(87) International publication number: WO 2001/044807

(56) References cited:
- JP-A- 2001 354 564
- KAUL SUNIL C ET AL: "An N-terminal region of mot-2 binds to p53 in vitro" NEOPLASIA (NEW YORK), vol. 3, no. 2, March 2001 (2001-03), pages 110-114, XP009026822 ISSN: 1522-8002
- WADHWA RENU ET AL: "Selective toxicity of MKT-077 to cancer cells is mediated by its binding to the hsp70 family protein mot-2 and reactivation of p53 function" CANCER RESEARCH, vol. 60, no. 24, 15 December 2000 (2000-12-15), pages 6818-6821, XP002271905 ISSN: 0008-5472
- WADHWA R ET AL: "Mortalin: A potential candidate for biotechnology and biomedicine." HISTOLOGY AND HISTOPATHOLOGY, vol. 17, no. 4, October 2002 (2002-10), pages 1173-1177, XP009026823 ISSN: 0213-3911
- DATABASE EBI [Online] mortalin sequence, 23 December 2003 (2003-12-23) "heat shock 70kDa protein" retrieved from EBI Database accession no. NP_004125 XP002271906
- WADHWA RENU ET AL: "The anti-proliferative aspects of mortalin (Review)" INTERNATIONAL JOURNAL OF ONCOLOGY, vol. 7, no. 1, 1995, pages 69-74, XP009026813 ISSN: 1019-6439
- WADHWA RENU ET AL.: 'Inactivation of tumor suppressor P53 by MOT-2, a HSP70 family member' THE JOURNAL OF BIOLOGICAL CHEMISTRY vol. 273, no. 45, 1998, pages 29586 - 29591, XP002937483
- KAUL SUNIL C. ET AL.: 'Inactivation of P53 and life span extension of human diploid fibroblasts by MOT-2' FEBS LETTERS vol. 474, 2000, pages 159 - 164, XP002937484

## Description

### Technical Field

The present invention relates to methods of screening for therapeutic agents using interacting regions of p53 and mortalin.

### Background Art

p53 is firmly established as one of the key tumor suppressor genes (Levine, A. J. (1997) Cell, 88, 323-331; Metz, T. et al. (1995) Cell, 82, 29-36). Functional inactivation of p53 is a very common characteristic of tumors (Harvey, D. M. and Levine, A. J. (1991) Genes Dev., 5, 2375-2385; Hainaut, P. et al. (1997) Nucleic Acids Res., 25, 151-157; Hollstein, M. et al. (1998) Mutat. Res., 405, 145-154; Moll, U. M. and Schramm, L. M. (1998) Critical Reviews In Oral Biol. Med., 9, 23-37) and the mechanisms whereby this occurs include the following three main categories: (i) mutations of p53 that abrogate its DNA binding or transcriptional activation functions; (ii) abnormal expression of p53-interacting proteins, e.g. mdm2, that result in accelerated degradation of the wild-type p53 or stability of mutant p53; and (iii) nuclear exclusion of wild-type p53 (Bosari, S. et al. (1995) Am. J. Pathol., 147, 790-798; Iwaya, K. et al. (1995) Lab Invest., 72, 707-714; Ostermeyer, A. G. et al. (1996) Proc. Natl. Acad. Sci. USA, 93, 15190-15194; Kubbutat, M. H. et al. (1999) Cell Growth Differ., 10, 87-92; Stommel, J. M. et al. (1999) EMBO J., 18, 1660-1672; Unger, T. et al. (1999) EMBO J., 18, 1805-1814).

While the first two categories have been intensively investigated in the last decade, the third remains poorly understood. The functional domains of p53 include an amino-terminus transactivation domain, a sequence-specific DNA binding domain, a carboxy-terminus oligomerization/tetramerization domain, and a regulatory domain (Harris, C. C. (1996) J. Natl. Cancer Inst., 88, 1442-1455). Accumulating evidence indicates that the activity of p53 is largely dependent on its conformation (Levine, A. J. (1997) Cell, 88, 323-331; Thomas, M. et al. (1999) Mol. Cell. Biol., 19, 1092-1100). The intracellular localization of p53 has been shown to be determined by nuclear localization signals; single amino acid residues, such as Leu³⁰⁵, Arg³⁰⁶ and Ser³¹⁵ (Liang, S. H. and Clarke, M. F. (1999) Oncogene, 18, 2163-2166); nuclear export or import signals (Middeler, G. et al.(1997) Oncogene, 14, 1407-1417; Stommel, J. M. et al. (1999) EMBO J., 18,1660-1672); and interactions of p53 with other proteins, including mdm2 and members of the hsp70 family (Hansen, S. et al. (1996) J. Biol. Chem., 271, 30922-30928; Selkirk, J. K. et al. (1996) Electrophoresis, 17, 1764-1771; Adler, V. et al. (1997) Proc. Natl. Acad. Sci. USA, 94, 1686-1691; Levine, A. J. (1997) Cell, 88, 323-331; Kamijo, T. et al. (1998) Proc. Natl. Acad. Sci. USA, 95, 8292-8297; Zhang, Y. et al. (1998) Cell, 92, 725-734; Tao, W. and Levine, A. J. (1999) Proc. Natl. Acad. Sci. USA, 96, 3077-3080).

Some mutant p53 proteins have been found in stable physical association with molecular chaperones, including hsp70, hsp90, cyclophilin 40, and p23 types that stabilize the conformation of the protein (Hinds, P. W. et al. (1987) Mol. Cell. Biol., 7, 2863-2869; Hainaut, P. and Milner, J. (1992) EMBO J., 11, 3513-3520; Lane, D. P. et al. (1993) Philos. Trans. R. So. Lond. B Biol. Sci., 339, 369-372; Merrick, B. A. et al. (1996) Biochim. Biophys. Acta , 13 , 57-68 ; Whitesell, L. et al. (1998) Mol. Cell. Biol., 18, 1517-1524). However, other mutant proteins, such as p53-273H and 281G, do not bind to hsc70, and thus it is not clear whether the complex formation between mutant p53 and hsc70 is required for the transforming function of mutant p53 (Hinds et al., 1990). Further, it has been proposed that hsc70 may facilitate cellular transformation by sequestering wild-type p53 protein (Hinds, P. W. et al. (1990) Cell Growth Differ., 1, 571-580). Recently, the present inventor has demonstrated that mot-2, a member of the hsp70 family, interacts with wild-type p53 (Wadhwa, R. et al. (1998) J. Biol. Chem., 273, 29586-29591).

Mortalins, mot-1 and mot-2, were cloned from normal and immortal mouse cells, respectively (Wadhwa, R. et al. (1993) J. Biol. Chem., 268, 6615-6621; Wadhwa, R. et al. (1993) J. Biol. Chem., 268, 22239-22242). Mortalin has also independently identified as PBP-74, mtHSP70, and Grp75; and has been assigned roles in antigen processing, *in vivo* nephrotoxicity, and radio-resistance in studies by other groups (Kaul, S. C. et al. (1998) Ind. J. Exp. Biol., 36, 345-352). Co-localization of mot-2 and wild-type p53 was observed in many transformed cells. Mot-1 that differs from mot-2 by only two amino acids in the carboxy-terminus and has pan-cytoplasmic cellular distribution Bhattachargya et al, JBC (1995), vol 270, pages 1705-1710 in normal cells that did not show any overlap with p53 according to immunostaining assay. Mot-2, but not mot-1, was found to inactivate wild-type p53 (Wadhwa, R. et al. (1998) J. Biol. Chem., 273, 29586-29591).

### Disclosure of the Invention

The object of the present invention is to identify regions contributing to the interaction between p53 and mortalin, to thereby provide methods of screening for drugs, particularly cancer therapeutic agents, by utilizing these regions.

As a result of conducting *in vitro* binding assays of p53 and mortalin in order to solve the above problems, the inventor of the present invention discovered that mortalin proteins, mot-1 and mot-2, have an equal p53 binding ability, and that the region of amino acid residues 253-282 of the mortalin protein is involved in the binding between mortalin and p53. In addition, according to the result of *in vivo* assay using a p53 responsive reporter gene, the mot-2 protein alone was demonstrated to remarkably inhibit p53-dependent transcription activation. On the basis of the results of analyses using various mutant mortalin proteins, the interaction between the mot-1 protein and p53 protein in cells was suggested to be restricted by a secondary structure unique to mot-1 or the presence of a third protein that specifically interacts with mot-1. According to the results of the *in vitro* binding assay, the region of the p53 protein that interacts with mortalin was mapped to the C-terminal amino acid residues 312-352 of the p53 protein. A domain involved in cytoplasmic sequestration of the p53 protein is included in this region, which domain is thought to contribute to the inhibition of the migration of p53 protein into the nucleus as reported in breast cancer, neuroblastoma, and colon cancer. The above results illustrate a new mechanism by which cancer cells inactivate p53 protein, the product of a major cancer inhibitory gene. By determining the interaction between p53 protein and mortalin protein as well as the regions within each protein that are involved in this interaction, a novel method of screening for cancer therapeutic agents is particularly provided.

The present invention relates to methods for screening cancer therapeutic agents using the interacting regions of p53 and mortalin, and more specifically, the present invention provides:
(1) a method of screening for candidate compounds of cancer therapeutic agents comprising the steps of:
   (a) contacting a peptide comprising amino acid residues 253 to 282 of an amino acid sequence of human mot-1 protein with a peptide comprising amino acid residues 312 to 352 of an amino acid sequence of human p53 in the presence of a test sample;
   (b) detecting binding between said peptides; and
   (c) selecting the compound that weakens the binding between said peptides, in comparison to binding that occurs in the absence of the test sample;
(2) a method of screening for candidate compounds of cancer therapeutic agents comprising the steps of:
   (a) contacting a test sample with a cell transformed with the following vectors:
      (i) a vector containing a DNA encoding a peptide comprising amino acid residues 253 to 282 of an amino acid sequence of human mot-1 protein;
      (ii) a vector containing a DNA encoding a peptide comprising amino acid residues 312 to 352 of an amino acid sequence of human p53, wherein said peptide activates human p53 responsive promoter; and
      (iii) a vector containing human p53 responsive promoter and a reporter gene functionally bound downstream thereof;
   (b) detecting the activity of said reporter gene within the cell; and
   (c) selecting the compound that increases the reporter expression, in comparison to that occurring in the absence of the test sample;
(3) a kit for screening candidate compounds of cancer therapeutic agents comprising:
   (a) a peptide comprising amino acid residues 253 to 282 of an amino acid sequence of human mot-1 protein; and
   (b) a peptide comprising amino acid residues 312 to 352 of an amino acid sequence of human p53;
(4) a kit for screening candidate compounds of cancer therapeutic agents comprising:
   (a) a vector containing a DNA encoding a peptide comprising amino acid residues 253 to 282 of an amino acid sequence of human mot-1 protein;
   (b) a vector containing a DNA encoding a peptide comprising amino acid residues 312 to 352 of an amino acid sequence of human p53, wherein said peptide activates human p53 responsive promoter; and
   (c) a vector containing human p53 responsive promoter and a reporter gene functionally bound downstream thereof;
(5) a compound isolated by the method of (1) or (2);
(6) a pharmaceutical composition comprising the compound of (5) as an active ingredient; and
(7) the pharmaceutical composition of (6), wherein the composition is a cancer therapeutic agent.

The present invention provides methods of screening for candidate compounds of cancer therapeutic agents by utilizing the interacting regions of p53 and mortalin protein. The screening of the present invention has various advantages over a screening that utilizes full-length proteins. For example, since peptides shorter than full-length proteins are used for the screening, the preparation is easier and more economical. In addition, stronger activities can be detected during the screening than in detected when the full-length proteins are used, which further enables more efficient screening. Moreover, development of drugs with high specificity is expected due to the use of interacting regions.

One embodiment of the method for screening of the present invention uses the binding of the interacting regions of p53 and mortalin protein as an index. More specifically, the method comprises: (a) contacting a peptide comprising the amino acid residues 253 to 282 of the amino acid sequence of human mortalin protein with a peptide comprising the amino acid residues 312 to 352 of the amino acid sequence of human p53 in the presence of a test sample; (b) detecting the binding between these peptides; and (c) selecting the compound that weakens the binding between these peptides, in comparison to that occurring in the absence of the test sample.

There is no particular limitation on the test sample to be used and it may include, for example, cell culture supernatants, fermenting bacterial products, marine organism extracts, plant extracts, prokaryotic cell extracts, eukaryotic unicellular extracts, animal cell extracts or their libraries, purified or crude proteins, peptides, non-peptide compounds, synthetic low molecular weight compounds, and natural compounds.

The mortalin peptide for use in the present invention, to be contacted with a test sample, is not restricted in any way so long as it includes the amino acids residues 253 to 282 of the amino acid sequence of human mot-1 protein (this region is common between human mot-1 protein and human mot-2 protein). Preferably the peptide comprises 600 amino acid residues or less of the amino acid residues of these proteins, more preferably 300 amino acid residues or less, and still more preferably 100 amino acid residues or less (e.g., 50 amino acid residues or less). Further, no particular limitations exist for the p53 peptide for use in the present invention, so long as it includes the amino acid residues 312 to 352 of the amino acid sequence of human p53. Preferably the peptide comprises 300 amino acid residues or less of the amino acid residues of these proteins, more preferably 200 amino acid residues or less, and still more preferably 100 amino acid residues or less (e.g., 50 amino acid residues or less). Preferably, at least one of the peptides used in the screening of the present invention is not a full-length protein. These peptides may be of natural origin, or may be prepared in accordance with known peptide synthesis methods.

In addition, these peptides can be contacted with a test sample, for example, in the form of purified peptides, solubilized peptides, peptides bound to a carrier, or peptides fused to other peptides.

Preparation of these peptides as recombinant peptides can be specifically carried out as described below. A gene encoding the target peptide is inserted into an exogenous gene expression vector, such as pSV2neo, pcDNA3, or pCD8, to express the gene in animal cells and so on. Any generally used promoters may be used for the expression including, for example, the SV40 early promoter (Rigby In Williamson (ed.), Genetic Engineering, Vol. 3, Academic Press, London, p. 83-141 (1982)), the EF-1α promoter (Kim et al., Gene 91, p. 217-223 (1990)), the CAG promoter (Niwa et al., Gene 108, p. 193-200 (1991), the RSV LTR promoter (Cullen, Methods in Enzymology 152, p. 684-704 (1987)), the SRα promoter (Takebe et al., Mol. Cell. Biol. 8, p. 466 (1988)), the CMV immediate early promoter (Seed and Aruffo, Proc. Natl. Acad. Sci. USA 84, p. 3365-3369 (1987)), the SV40 late promoter (Gheysen and Fiers, J. Mol. Appl. Genet. 1, p. 385-394 (1982)), the Adenovirus late promoter (Kaufman et al., Mol. Cell. Biol. 9, p. 946 (1989)), the HSV TK promoter, and so on.

Examples of methods for introducing an exogenous gene into animal cells so as to allow for expression of the gene include the electroporation method (Chu, G. et al., Nucl. Acids Res. 15, p. 1311-1326 (1987)), the calcium phosphate method (Chen, C. and Okayama, H., Mol. Cell. Biol. 7, p. 2745-2752 (1987)), the DEAE dextran method (Lopata, M. A. et al., Nucl. Acids Res. 12, p. 5707-5717 (1984); Sussman, D. J. and Milman, G., Mol. Cell. Biol. 4, p. 1642-1643 (1985)), the Lipofectin method (Derijard, B., Cell 7, p. 1025-1037 (1994); Lamb, B. T. et al., Nature Genetics 5, p. 22-30 (1993); Rabindran, S. K. et al., Science 259, p. 230-234 (1993)), and so on; however, any suitable method may be used. The peptides of the present invention can be expressed as fusion peptides, having a recognition site for a monoclonal antibody, by introducing a recognition site (epitope) for a monoclonal antibody with a pre-determined specificity into the N-terminus or C-terminus of a peptide of the present invention. Commercially available epitope-antibody systems may be used (Experimental Medicine 13, p. 85-90 (1995)). Vectors expressing fusion peptides, for example, with β-galactosidase, maltose binding protein, glutathione S-transferase, green fluorescent protein (GFP), and such, via a multi-cloning site are commercially available.

In order to minimize changes in the properties of the peptides of the present invention as a result of preparing them in the form of a fusion peptide, a method has been reported wherein the fusion peptides are prepared by introducing only a small epitope portion that comprise several to dozens of amino acids. Examples include epitopes such as polyhistidine (His-tag), influenza agglutinin HA, human c-myc, FLAG, vesicular stomatitis virus glycoprotein (VSV-GP), T7 gene 10 protein (T7-tag), human Herpes simplex virus glycoprotein (HSV-tag), and E-tag (epitope on a monoclonal phage); these epitopes can be used together with monoclonal antibodies that recognize the epitopes as an epitope-antibody system for screening proteins that bind with the peptides of the present invention (Experimental Medicine 13, p. 85-90 (1995)).

The screening system provided by the present invention can be carried out as an *in vitro* assay system. Specific examples include *in vitro* assay systems carried out in a non-cellular system. More specifically, a screening that uses the binding of the above peptide pairs as an index can be carried out by binding either of the peptides to a support, and adding the other peptide and a test sample thereto, incubating the mixture followed by washing and detection or measurement of the binding of the other peptide to the peptide bound to the support.

The peptides of the present invention may be used as purified or roughly purified peptides produced from cells that endogenously express the peptides, cells transformed with a DNA that encodes the peptides, and animals or plants transformed with a DNA that encodes the peptides.

The peptides used in the present invention can be bound to a support. For example, first, one of the two peptides, which is purified or roughly purified, is bound to a support. The peptide may be bound to a support by conventional methods to fix peptides to a support. Examples of supports that may be used to bind the peptides include insoluble polysaccharides, such as agarose, dextran, and cellulose; synthetic resins, such as polystyrene, polyacrylamide, and silicon; and so on. More specifically, commercially available beads and plates manufactured from the above raw materials may also be used. For the use of beads, a column may be filled with the beads. Examples of plates that may be used include multi-well plates (96-well multi-well plates) and biosensor chips.

In order to bind peptides to a support, routine methods utilizing chemical bonding, physical adsorption, and such may be used. In addition, antibodies that specifically recognize the peptide can be bound to the support in advance, and then, the peptides may be bound to the support via the antibodies. Moreover, binding of a peptide and a support can be also carried out by means of avidin/biotin binding.

Binding between peptides is usually carried out in a buffer. Examples of buffers include phosphate buffer, Tris buffer, and so on. The condition for incubation include such conditions already well known in the art, for example, from 4°C to room temperature for 1 to 24 hours. Washing after incubation may be carried out with any solutions so long as the binding of peptides is not inhibited, and can be exemplified by buffer containing surfactants. An example of the surfactant is 0.05% Tween 20.

In selecting the desired compound, specific binding and non-specific binding can be distinguished by incubating each of the peptides and the test sample under suitable conditions followed by washing. Either of the peptides may be bound to the support for selecting the desired compound. For example, when a peptide derived from mortalin protein is bound to a support, after fixing the peptide, a pre-mixed mixture of peptide derived from p53 protein and a test sample may be added to the support, or peptide derived from p53 protein may be added following the addition of a test sample. Similarly, in the case of fixing the peptide derived from p53 protein, a pre-mixed mixture of peptide derived from mortalin protein and a test sample may be added to the fixed peptide, or peptide derived from mortalin protein may be added following the addition of a test sample. Then, the state of the binding between peptides can be evaluated by incubating the peptides and test sample added in the above order under suitable conditions.

According to the screening method of the present invention, a control group may be adopted together with a "test" group, wherein test samples are contacted with the peptides. A negative control group that does not contain any test sample, a positive control group, or both may be used in the screening method of the present invention.

In the present invention, during detection or measurement of bound peptides, the bound peptide may be either directly detected or measured quantitatively. The desired compound can be detected by comparing the result obtained for a negative control group containing no test sample and the result obtained for the group containing the test sample and/or the results obtained for a positive control group.

In addition, these results may be obtained as numerical values, and comparing those numerical values enables quantitative measurement of the activity of the desired compound. For a quantitative measurement, the desired compound may be detected by comparing the numerical value obtained with a negative control group containing no test sample with the numerical value obtained with a group including the test sample. If the resulting numerical values decrease in comparison with the negative control group, the test sample is judged to contain the desired compound.

Further, a quantitative measurement can be conducted by using a standard curve prepared based on numerical values obtained with a positive control group containing known amounts of compound that is known to inhibit the binding between the peptides. The activity of a compound to inhibit peptide binding is judged to be weak when large amount of peptides are bound; on the other hand, when the amount of bound peptide is small, the inhibitory activity of the compound to inhibit binding of the peptide is judged to be strong.

In the present invention, a biosensor utilizing the surface plasmon resonance phenomenon can be used as means for detecting or measuring bound peptides. Biosensors utilizing the surface plasmon resonance phenomenon enable real-time observation of peptide-peptide interaction by generating a surface plasmon resonance signal, even with an extremely small amount of peptide and without requiring labeling of the peptides (e.g., BIAcore, Pharmacia). Thus, the use of BIAcore or other biosensor enables real-time evaluation of the binding of the peptides of the present invention.

Specifically, first, one of the two peptides used in the screening of the present invention is fixed on a sensor chip; the other peptide is contacted to the sensor chip. The other peptide binding to the peptides on the sensor chip are detected as a change in the resonance signal.

More specifically, the above method may be carried out as described below. First, CM5 sensor chip (Biosensor) is activated to fix either the peptides derived from p53 or those derived from mortalin protein on the sensor chip. More specifically, the sensor chip is activated with aqueous EDC/NHS solution (containing 200 mM EDC (N-ethyl-N'-(3-dimethylaminopropyl) carbonate hydrochloride) and 50 mM NHS (N-hydroxysuccinimide)), and then, is washed with HBS buffer (containing 10 mM HEPES pH 7.4, 150 mM NaCl, 3.4 mM EDTA and 0.05% Tween 20).

Next, a suitable amount of the first peptide with interacting ability dissolved in HBS buffer is contacted with the sensor chip to fix the peptides thereon. After washing the sensor chip with HBS buffer, residual activity group of the sensor chip is blocked with ethanolamine solution (1 M ethanolamine hydrochloride, pH 8.5). Then, the sensor chip is washed again with HBS buffer for the use in binding evaluation.

Next, a suitable amount of the second peptide (the other peptide of the two peptides used in the screening of the present invention, which was not bound to the sensor chip) dissolved in HBS buffer is injected. The amount of the second peptide having an ability to interact, bind, with the peptide fixed on the sensor chip is observed as an increase in the value of the resonance signal at the time of addition.

Then, a test sample is injected following the injection of the second peptide that interacts with the first peptide in the above binding evaluation system. In addition, a control group may be adopted together with the test group wherein a test sample is injected. The control group may include a negative control group that lacks the test sample, a positive control group that includes the test sample, or both.

The bound peptide can be measured quantitatively as a degree of change in the resonance signal value. The desired compound can be detected and measured by comparing results obtained for a negative control group that contain no test sample with results obtained for a group that contain the test sample and/or results obtained for a positive control group.

In the present invention, either of the two peptides may be labeled, and the label of the bound peptide may be used to detect or measure the bound peptide.

For example, in the above-mentioned screening method, after pre-labeling the second peptide that is contacted with the other peptide together with a test sample, the peptides are incubated with the test sample, and then, bound peptides are detected or measured according to the label after washing. Specifically, a test sample and a labeled peptide is contacted with a peptide that is preferably bound to a support. After incubation and washing, the label of the peptide bound on the other peptide can be detected and measured.

The peptides used in the present invention can be labeled by generally known methods. Example of labeling substance includes radioisotopes, enzymes, fluorescent substances, biotin/avidin, and so on. Commercially available labeling substances can be used as such labeling substances. Examples of radioisotopes include ³²P, ³³P ¹³¹I, ¹²⁵I, ³H, ¹⁴C, and ³⁵S. Examples of enzymes include alkaline phosphatase, horseradish peroxidase, β-galactosidase, β-glucosidase, etc. Examples of florescent substances include fluorescein isothiocyanate (FITC) and rhodamine. Such labeling substances are commercially available products, and can be used to label substances in accordance with known methods.

More specifically, the labeling can be carried out as described below. Namely, a solution containing either of the peptides is added to a plate and allowed to stand overnight. After washing the plate, blocking, for example, with BSA is performed to prevent non-specific peptide binding. After washing the plate again, a test sample and the other peptide that which was not fixed on the plate yet has been labeled, is added to the plate. At the same time, a negative control group containing no test sample and/or a positive control group are provided and are also incubated. After the incubation, the plate is washed to detect or measure the bound peptide. The bound peptide may be detected or measured by liquid scintillation of a radioisotope. Alternatively, in the case where an enzyme is used as the label, a substrate of the enzyme is added, and the enzymatic change of the substrate, such as the generation of color, may be detected or measured using absorptiometer. Further, when a fluorescent substance is used as the label, the bound peptide may be detected or measured using fluorophotometer. Then, the desired compound can be determined by comparing these results with the numerical values obtained for a control group.

In the present invention, antibodies specifically recognizing either of the two peptides can be used as means for detecting or measuring the bound peptide.

For example, after contacting one of the two peptides with a test sample and the other peptide, the material is incubated and washed, and the bound peptide is detected or measured through a primary antibody that specifically recognizes the peptide. Specifically, the test sample and the other peptide are contacted with the peptide fixed on a support. Following incubation and washing, the bound peptide can be detected or measured with a primary antibody that specifically recognizes the bound peptide. The primary antibody is preferably labeled with a labeling substance.

More specifically, the method can be carried out as described below. Namely, a solution containing one of the two peptides is added to a plate and allowed to stand overnight. After washing the plate, blocking, for example, with BSA is conducted to prevent non-specific peptide binding. After washing the plate again, a test sample and the other peptide are added to the plate. At the same time, a negative control group containing no test sample and/or a positive control group are provided, and are also incubated.

Following incubation and washing, primary antibody against the peptide added together with the test sample is added. After suitable incubation, the plate is washed and the bound peptide is detected or measured by the primary antibody. For detection or measurement when the primary antibody is labeled with a radioisotope, the bound peptide may be subjected to liquid scintillation. In the case where the label is an enzyme, an enzyme substrate is added and the enzymatic change in the substrate, such as generation of color, may be detected or measured using absorptiometer. When using a fluorescent substance as the label, the bound peptide may be detected or measured using fluorophotometer. Then, the desired compound can be determined by comparing these results with the numerical values obtained for a control group.

Further, a primary antibody specifically recognizing peptides that are fused to the peptides used in the present invention can be utilized as means for detecting or measuring the bound peptide in the present invention.

For example, according to the above-mentioned screening method, after contacting and incubating either of the two peptides (e.g., the first peptide) used in the method of the present invention with a test sample and the other peptide (e.g., the second peptide), washing is conducted, and the second peptide that is bound to the first peptide is detected or measured by a primary antibody that specifically recognizes a peptide fused to the second peptide. Specifically, the test sample and the second peptide are contacted with the first peptide preferably fixed on a support. Following incubation and washing, the second peptide that is bound to the first peptide is detected or measured by a primary antibody that specifically recognizes the peptide fused to the second peptide. The primary antibody is preferably labeled with a labeling substance.

More specifically, the method is carried out as described below. Namely, a solution containing either of the two peptides (first peptide) used in the present method is added to a plate and allowed to stand overnight. After washing the plate, blocking, for example, with BSA is conducted to prevent non-specific peptide binding. After washing the plate again, a test sample and the other peptide (second peptide) fused with another peptide (third peptide) are added to the plate. At the same time, a negative control group containing no test sample and/or a positive control group are provided and are incubated.

Following incubation and washing, antibodies to the third peptide fused to the second peptide that was added together with the test sample are added. After suitable incubation, the plate is washed and the second peptide is detected or measured by a primary antibody that specifically recognizes the third peptide fused to the second peptide. The detection or measurement of the second peptide may be conducted by liquid scintillation when the primary antibody is labeled with a radioisotope. In the case where the label is an enzyme, enzyme substrate is added and the enzymatic change of the substrate, such as the generation of color, may be detected or measured using absorptiometer. In the case of a fluorescent substance as the label, the second peptide may be detected or measured using fluorophotometer. Then, the desired compound can be determined by comparing these results with numerical values obtained for a control group.

In the present invention, a primary antibody that specifically recognizes the peptides used in the present invention and a secondary antibody that specifically recognizes the primary antibody can be used as means for detecting or measuring the bound peptides.

For example, after contacting one of the two peptides (first peptide) with the other peptide (second peptide) together with a test sample, the mixture is incubated and washed, and the second peptide bound to the first peptide is detected or measured with primary antibodies that specifically recognize the second peptide and secondary antibodies that specifically recognize the primary antibody. Specifically, the test sample and the second peptide are contacted with the first peptide preferably bound to a support. Following incubation and washing, the second peptide bound to the first peptide can be detected or measured with a primary antibody that specifically recognizes the second peptide and a secondary antibody that specifically recognizes the primary antibody. The secondary antibody is preferably labeled with a labeling substance.

More specifically, the method can be carried out as described below. Namely, a solution containing either of the two peptides (first peptide) is added to a plate and allowed to stand overnight. After washing the plate, blocking, for example, with BSA, is performed to prevent non-specific peptide binding. After washing the plate again, a test sample and the other peptide (second peptide) are added to the plate. At the same time, a negative control group containing no test sample and/or a positive control group are provided and incubated.

Following incubation, a primary antibody against the second peptide, which has been added together with the test sample, is added after washing. After suitable incubation, the plate is washed, and a secondary antibody is added, which antibody specifically recognizes the primary antibody. After suitable incubation and washing of the plate, the bound peptide is detected or measured by the secondary antibody that specifically recognizes the primary antibody which, in turn, specifically recognizes the second peptide. The detection or measurement may be conducted by liquid scintillation when a radioisotope is used as the label. In the case where the label is an enzyme, the enzyme substrate is added and the enzymatic change of the substrate, such as the generation of color, may be detected or measured using absorptiometer. In the case of a fluorescent substance as the label, the detection or measurement may be conducted using fluorophotometer. Then, the desired compound is selected by comparing these results with numerical values obtained for a control group.

In the present invention, a primary antibody that specifically recognizes another peptide fused to one of the two peptides used in the present invention, and a secondary antibody that specifically recognizes the primary antibody can be used as means for detecting or measuring the peptide bound to the other peptide.

For example, according to the above-mentioned screening method, after contacting one of the two peptides (first peptide) with the other peptide (second peptide) together with a test sample, incubation and washing is conducted, and the second peptide that is bound to the first peptide is detected or measured by a primary antibody that specifically recognizes another peptide (third peptide) fused to the second peptide and a secondary antibody that specifically recognizes the primary antibody. Specifically, the second peptide and the test sample are contacted with the first peptide that is preferably bound to a support. Following incubation and washing, the second peptide that is bound to the first peptide can be detected or measured by a primary antibody that specifically recognizes the third peptide fused to the second peptide, and a secondary antibody that specifically recognizes the primary antibody. The secondary antibody is preferably labeled with a labeling substance.

More specifically, the method can be carried out as described below. Namely, a solution containing either of the two peptides (first peptide) is added to a plate and allowed to stand overnight. After washing the plate, blocking, for example, with BSA, is performed to prevent non-specific peptide binding. After washing the plate again, a test sample and the other peptide (second peptide) fused with another peptide (third peptide) are added to the plate. At the same time, a negative control group containing no test sample and/or a positive control group are provided and incubated.

Following incubation, a primary antibody to the third peptide fused to the second peptide that has been added together with the test sample is added after washing. After suitable incubation, the plate is washed, and a secondary antibody is added, which antibody specifically recognizes the primary antibody. After suitably incubating and washing the plate again, the second peptide is detected or measured by a secondary antibody that specifically recognizes the primary antibody, wherein the primary antibody, in turn, specifically recognizes the third peptide fused to the second peptide. The detection or measurement may be performed by liquid scintillation wherein a radioisotope is used as the label. In the case of an enzyme as the label, enzyme substrate is added and the enzymatic change in the substrate, such as the generation of color, may be detected or measured using absorptiometer. In the case of a fluorescent substance as the label, the bound second peptide may be detected or measured using fluorophotometer. The desired compound can then be determined by comparing these results with numerical values obtained for a control group.

The detection or measurement is preferably carried out by Enzyme-Linked Immunoabsorbent Assay (ELISA) as described below. Namely, a peptide (first peptide) of the peptide pairs used in the present method, for example, any peptide fused with 6x His, is diluted with fixation buffer (0.1 M NaHCO₃, 0.02% NaN₃, pH 9.6). A suitable amount of the diluted solution is then added to each well of a 96-well Immunoplate (Nunc) followed by incubation overnight at 4°C.

After washing each well three times with washing buffer (prepared to contain 0.05% Tween 20 in PBS), 200 µl of a solution of 5% BSA (SIGMA) dissolved in PBS is added to perform blocking overnight at 4°C.

Next, after washing each well three times with washing buffer, suitable amounts of the other peptide (second peptide) of the present peptide pair diluted with dilution buffer (1% BSA, 0.5% Tween 20, and PBS), for example a peptide fused with FLAG, and a test sample are added and incubated for 1 hour at room temperature. After washing each well three times with washing buffer, 100 µl of mouse anti-FLAG antibody (IBI) diluted to 3 µg/ml with dilution buffer is added to each well followed by incubation for 1 hour at room temperature.

After washing each well three times with washing buffer, 100 µl of alkaline phosphatase-labeled goat anti-mouse IgG antibody (ZYMED) diluted 1000-fold with a dilution buffer is added to each well followed by incubation for 1 hour at room temperature. After washing each well five times with washing buffer, 100 µl of color developing solution (p-phenylphosphate (SIGMA) dissolved to a concentration of 1 mg/ml in substrate buffer (50 mM NaHCO₃, 10 mM MgCl₂, pH 9.8)) is added to each well, and after allowing to react at room temperature, absorbance at 405 nm is measured using microplate reader (Model 3550, BIO-RAD). The desired compound can then be determined by comparing these results with numerical values obtained for a negative control group and/or positive control group.

It should be noted that the detection or measurement using an antibody of the present invention can be also conducted by using protein G or protein A in place of the secondary antibody.

High throughput screening (HTS) may be utilized for the screening method of the present invention. More specifically, high throughput screening can involve manually performing the procedure until the blocking step, and then automatically performing the subsequent reactions, with the aid of a robot, for example.

Namely, the first peptide, for example a peptide fused to 6x His, is diluted with fixation buffer (0.1 M NaHCO₃, 0.02% NaN₃, pH 9.6). A suitable amount of this diluted aqueous solution is then added to each well of a 96-well Immunoplate (Nunc) followed by incubation overnight at 4°C.

After washing each well three times with washing buffer (prepared to contain 0.05% Tween 20 in PBS), 200 µl of a solution of 5% BSA (SIGMA) dissolved in PBS is added to perform blocking overnight at 4°C.

Next, the blocked Immunoplate is placed in, for example, Biomek 2000 HTS system (Beckman), followed by running a system control program. At this time, injection and removal of solution into and from each well can be carried out by Biomek 2000 (Beckman) or Multipipette 96 well simultaneous injector (Sagian) as the injector. In addition, EL404 Microplate Washer (Bio-Tek) can be used for washing each well of the immunoplate. Further, SPECTRAmax 250 plate reader (Molecular Devices) can be used for measuring absorbance.

The program is set up to perform the following procedure. Namely, after washing each well three times with washing buffer, suitable amounts of test sample and the second peptide, for example; a peptide that is fused with another peptide, such as MBP (maltose bound protein), that are diluted with a dilution buffer (1% BSA, 0.5% Tween 20, and PBS) are added. At the same time, a negative control group containing no test sample and a positive control group are provided, and are incubated for 1 hour at room temperature.

After washing each well three times with washing buffer, 100 µl of rabbit anti-MBP antiserum (New England Biolabs) diluted 5000-fold with dilution buffer are added to each well followed by incubation for 1 hour at room temperature. After washing each well again three times with washing buffer, 100 µl of alkaline phosphatase-labeled goat anti-rabbit IgG antibody (TAGO) diluted 5000-fold with dilution buffer are added to each well followed by incubation for 1 hour at room temperature.

After washing each well five times with washing buffer, 100 µl of color developing solution (p-nitrophenyl phosphate (SIGMA) dissolved to a concentration of 1 mg/ml in substrate buffer (50 mM NaHCO₃, 10 mM MgCl₂, pH 9.8)) is added to each well, and after allowing to react at room temperature, absorbance at 405 nm may be measured using microplate reader or Biomek plate reader (Beckman/Molecular Devices). Then, the desired compound is determined by comparing these results with numerical values obtained for the control groups.

Commercially available antibodies or antibodies contained in commercially available kits can be used as the antibody used in the methods of the present invention, and monoclonal antibodies or polyclonal antibodies obtained according to known procedures can also be used.

Primary antibodies or secondary antibodies can be labeled by known methods. Examples of labeling substances include radioisotopes, enzymes, and fluorescent substances. Commercially available labeling substances can be used for these labeling substances. Examples of radioisotopes include ³²P, ³³P, ¹³¹I, ¹²⁵I, ³H, ¹⁴C, and ³⁵S. Examples of enzymes include alkaline phosphatase, horseradish peroxidase, β-galactosidase, and β-glucosidase. Examples of fluorescent substances include fluorescein isothiocyanate (FITC) and rhodamine.

In addition, detection of peptide binding can be carried out by detecting and/or measuring changes in the amount of reporter gene expressed, whose expression is activated in response to the binding of the peptides. Examples of such reporter genes include luciferase, β-galactosidase, HIS3 gene, chloramphenicol-acetyl transferase (CAT), and green fluorescence protein (GFP) gene.

The peptides expressed in cells may be peptides fused with other peptides. Although the other peptide used for the fusion with the peptides expressed in cells may be any peptide so long as it can be used in the screening method of the present invention, it is preferably a transcription regulatory factor.

For example, DNAs encoding two different peptides (the binding of the two peptide is measured), respectively, wherein the peptides respectively are fused with subunits of a transcription regulatory factor that is a heterodimer known to activate transcription of a reporter gene by binding to a DNA, can be constructed and introduced into expression vectors to transform cells. When the test sample does not contain any compounds inhibiting the binding of the peptides, the two peptides form a heteromer, i.e. the transcription regulatory factor, binding to the DNA to activate the reporter gene.

On the other hand, when a compound that inhibits the binding of the peptides is contained in the test sample, the binding between the peptides is inhibited and as a result, the subunits of the transcription regulatory factor are unable to form the heteromer needed to induce transcription of the reporter gene. Then, the desired compound can be detected or measured by determining the change in the amount of reporter gene expressed. To determine the change in the amount of the reporter gene expressed in this type of system, a two-hybrid system ("Two-hybrid system" Fields, S., and Sternglanz, R., Trends. Genet. (1994) 10, 286-292) or three-hybrid system can be used.

The hybrid system can be constructed by conventional methods or using commercially available kits. Examples of commercially available two-hybrid system kits include MATCHMAKER Two-Hybrid System, Mammalian MATCHMAKER Two-Hybrid Assay Kit (both made by CLONTECH), and HybriZAP Two-Hybrid Vector System (Stratagene).

More specifically, a two-hybrid system can be constructed as described below. Namely, a gene that codes for either of the two peptides (e.g., the first peptide) and a gene that codes for the DNA binding domain of LexA are linked to produce an expression vector. Herein, the expression plasmid is constructed by inserting the desired gene fragment into a yeast two-hybrid expression plasmid pBTM116 (Vojtek, A.B., et al., Cell (1993) 74, 205-214).

Then, an expression vector is prepared by linking a gene encoding the other peptide (e.g., the second peptide) to a gene that encodes the transcription activating domain of GAL4. Yeast two-hybrid expression plasmid pGAD10 (Clontech) can be used for the expression vector.

After transforming yeast strain L40 incorporated with HIS3 gene, which transcription is regulated by a promoter containing a LexA binding motif, with both two-hybrid expression plasmids, the growth of the yeast will be only observed when peptide interaction occurs following the incubation on histidine-free synthetic medium. Thus, an increase in the amount of expressed reporter gene can be determined according to the degree of the growth of the transformant, thereby enabling the screening of the desired compound.

In addition, another embodiment of the screening method of the present invention relates to a method that uses a construct containing the p53 responsive promoter and a reporter gene expressed in response to the activation of the promoter. More specifically, the method comprises the steps of:
(a) contacting a test sample with a cell transfected with the following vectors:
   (i) a vector comprising a DNA encoding a peptide comprising the amino acid residues 253 to 282 of the amino acid sequence of human mot-1 protein or human mot-2 protein;
   (ii) a vector comprising a DNA encoding a peptide comprising the amino acid residues 312 to 352 of the amino acid sequence of human p53 wherein the peptide has the function of activating human p53 responsive promoter; and
   (iii) a vector comprising a reporter gene functionally bound downstream of human p53 responsive promoter;
(b) detecting the reporter activity within the cells; and
(c) selecting the compound that increases the reporter activity, in comparison to that occurring in the absence of the test sample.

According to the method, first, a vector that expresses human mortalin, a vector that expresses human p53, and a vector that expresses a reporter gene in response to the expression of human p53 are constructed and introduced into cells.

The human mortalin expressed by the vector is not particularly restricted and any peptide can be used so long as the peptide includes the domain for interaction with p53 (i.e., amino acid residues 253 to 282). Preferably the peptide contains 600 amino acid residues or less of the amino acid residues of these proteins, more preferably 300 amino acid residues or less, and even more preferably 100 amino acid residues or less (for example, 50 amino acid residues or less). On the other hand, there are no particular restrictions on the human p53 expressed by the vector so long as it includes the domain that interacts with mortalin (i.e., amino acid residues 312 to 352). Preferably, the peptide contains 300 amino acid residues or less of the amino acid residues of these proteins, more preferably 200 amino acid residues or less, and even more preferably 100 amino acid residues or less (for example, 50 amino acid residues or less).

These vectors can be constructed, for example, by inserting a DNA encoding these peptides into a mammalian expression vector, such as pSG5 or pcDNA3. In addition, construction of a vector wherein the reporter gene is functionally bound downstream of human p53 responsive promoter can be carried out by inserting human p53 responsive promoter into an expression vector comprising the reporter gene. Herein, the phrase "functionally bound" refers to the binding of a promoter and reporter gene to enable expression of the reporter gene in response to the activation of human p53 responsive promoter. An example of human p53 responsive promoter that can be used in the present invention is described in the literature (El-Deiry, W.S., et al., (1993) Cell, 75, 817-825). In addition, any reporter gene may be used so long as its expression can be detected; examples include β-gal gene, the CAT gene, and the luciferase gene. Although there are no particular restrictions on the cells into which a vector is introduced, preferable examples include MCF-7 cells and NIH3T3 cells.

According to the present invention, a test sample is contacted with the cells prepared as above. There are no particular restrictions on the test sample; examples include cell culture supernatants, fermenting microorganism products, marine organism extracts, plant extracts, prokaryotic cell extracts, eukaryotic unicellilar extracts, animal cell extracts or their libraries, purified or crude proteins, peptides, non-peptide compounds, synthetic low molecular weight compounds, and naturally-occurring compounds.

After contacting a test sample, the activity of the reporter in the cells is detected. Detection of the reporter activity can be carried out corresponding to the type of reporter gene by methods known to those skilled in the art. As a result of detecting the reporter activity, if the reporter activity detected in the cells contacted with the test sample increases significantly as compared with the result obtained without a test sample (control), the used test compound serves as a candidate for a compound that inhibits the interaction between mortalin protein and p53.

The present invention also relates to a kit for carrying out the above described screening method, which comprises: (a) a peptide comprising the amino acid residues 253 to 282 of the amino acid sequence of human mot-1 protein or human mot-2 protein; and (b) a peptide comprising the amino acid residues 312 to 352 of the amino acid sequence of human p53.

Moreover, the present invention also relates to a kit for carrying out the above described screening method, which comprises: (a) a vector comprising a DNA encoding a peptide comprising the amino acid residues 253 to 282 of the amino acid sequence of human mot-1 protein or human mot-2 protein; (b) a vector comprising a DNA encoding a peptide that comprises the amino acid residues 312 to 352 of the amino acid sequence of human p53, which peptide has the function to activate human p53 responsive promoter; and (c) a vector comprising a reporter gene functionally bound downstream of human p53 responsive promoter. The kit may additionally contain cells for introducing these vectors.

In addition, the present invention also provides compounds isolated by the screening methods of the present invention and pharmaceutical applications thereof. p53 is established as a tumor suppressor gene (Levine, A.J. (1997) Cell, 88, 323-331; Metz, T. et al. (1995) Cell, 82, 29-36). Consequently, compounds isolated by the screening methods of the present invention serve as important candidates for cancer therapeutic agents.

The compounds isolated by the screening methods of the present invention can be used as pharmaceutical agents, for example, for humans, mice, rats, guinea pigs, rabbits, chickens, cats, dogs, sheep, pigs, cows, monkeys, baboons, or chimpanzees, by directly administering the protein or the isolated compound itself, and they may be also administered in the form of a pharmaceutical composition by formulating in accordance with known pharmaceutical methods.

For example, they may be orally administered as tablets, which may be sugar-coated as necessary; capsules; elixirs; and microcapsules, or parenterally administered as injections, in the form of an aseptic solution or suspension with water or other pharmaceutically acceptable liquid. For example, a pharmaceutical composition may be formulated by mixing into a unit dose form required for typically observed pharmaceutical preparation by suitably combining with a pharmacologically acceptable carrier or medium, specific examples of which include sterilized water or physiological saline, vegetable oil, emulsifier, suspension agent, surfactant, stabilizer, flavoring, excipient, vehicle, antiseptic, binder, and so on. The amount of active ingredient in these preparations is adjusted so to obtain a suitable volume within a specified range.

Examples of additives that can be mixed into tablets and capsules include binders, such as gelatin, cornstarch, gum tragacanth, and gum arabic; excipients, such as crystalline cellulose; swelling agents, such as cornstarch, gelatin, and alginic acid; lubricants, such as magnesium stearate; sweeteners, such as sucrose, lactose, and saccharin; and flavorings, such as peppermint, *Gaultheria adenothrix* oil, and cherry flavoring. When the preparation unit is in the form of a capsule, liquid carriers, such as fats and oils, can be contained in addition to the above materials. Aseptic compositions for injection can be formulated in accordance with ordinary preparation formulation using vehicles, such as distilled water for injection.

Examples of aqueous solutions suitable for injection include physiological saline, and isotonic liquids containing glucose or other adjuvants, such as D-sorbitol, D-mannose, D-mannitol, and sodium chloride, and these adjuvants may be used in combination with suitable dissolving assistants, examples of which include alcohols, such as ethanol; polyalcohols including propylene glycol and polyethylene glycol; and nonionic surfactants, such as Polysorbate 80 (TM) and HCO-50.

Examples of oily liquids include sesame oil and soybean oil, and these may be used in combination with dissolving adjuvants, such as benzyl benzoate and benzyl alcohol. In addition, buffers, such as phosphate buffer and sodium acetate buffer; analgesics, such as procaine hydrochloride; stabilizers, such as benzyl alcohol and phenol; and antioxidants may also be blended. The prepared injection liquid is normally filled into suitable ampules.

Administration to patients can be carried out by methods known to those skilled in the art, for example, by intraarterial injection, intravenous injection or subcutaneous injection; or intranasal, transbronchial, intramuscular, percutaneous or oral administration. Although the dosage varies depending on the body weight and age of the patient, administration method, and so on, an appropriate dosage can be suitably selected by a person skilled in the art. In addition, if the compound can be encoded by a DNA, gene therapy may also be carried out by incorporating the DNA into a vector for gene therapy. Although the dosage and administration method vary according to the body weight, age, and symptoms of the patient, they can be suitably selected by a person skilled in the art.

Although the dosage of the compound of the present invention varies according to the symptoms, in the case of oral administration, the dosage for adult (the body weight as 60 kg) typically ranges from about 0.1 to 100 mg per day, preferably about 1.0 to 50 mg per day, and more preferably about 1.0 to 20 mg per day.

In the case of parenteral administration, although varying according to the subject for administration, target organ, symptoms, and administration method, the single dosage in the form of an injection preparation, for example, for an adult (a body weight of 60 kg) is normally about 0.01 to 30 mg per day, preferably about 0.1 to 20 mg per day, and more preferably about 0.1 to 10 mg per day; and the administration is preferably carried out by intravenous injection. For administration to other animals, an amount of the compound converted for the amount per 60 kg of body weight or the amount converted for the amount per body surface area can be administered.

### Brief Description of the Drawings

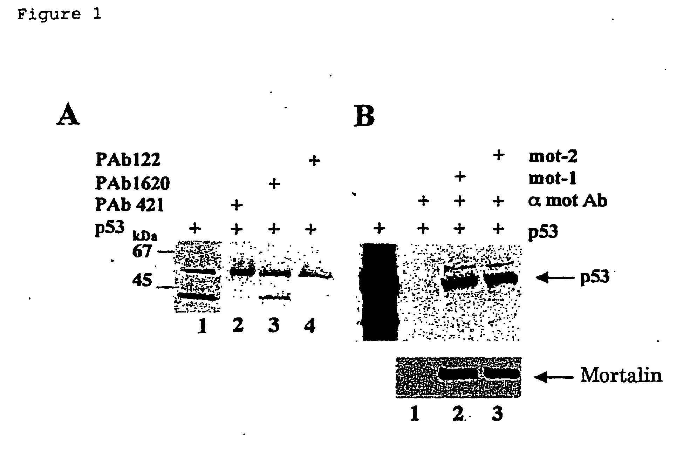

Figure 1 depicts photographs demonstrating the results of detecting *in vitro* binding of mot-1 and mot-2 to p53.
   (A) *In vitro* translated ³⁵S-labeled murine p53 was immunoprecipitated with anti-p53 antibodies. The small sized (43-kDa) product precipitated with PAb 1620 (specific for wild-type p53 conformation) but not with PAb 421 that recognizes a carboxy-terminal epitope.
   (B) p53 co-precipitated with both mot-1 and mot-2 (lanes 2-3), whereas the carboxy-terminal truncated form of p53 did not. Immunoprecipitated mortalins were detected by Western blotting with anti-His tag antibody and p53 was detected by autoradiography.

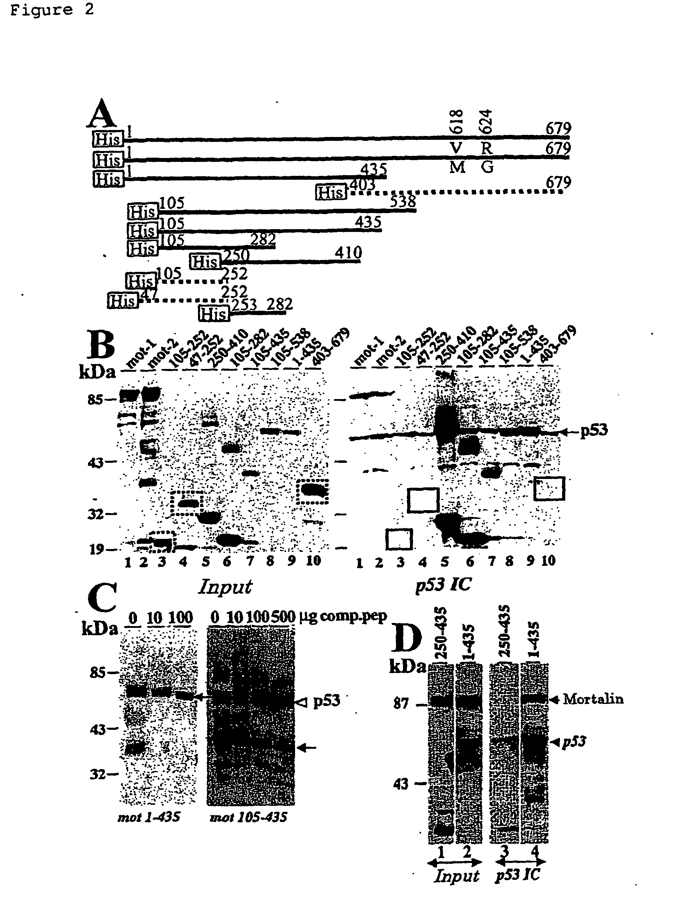

Figure 2 depicts drawings ((B)-(D) are photographs) demonstrating the mapping of the p53 binding domain of mortalins.
   (A) Schematic illustration of mortalin deletion mutants. The two amino acids (residues 618 and 624) that differ between mot-1 and mot-2 are indicated. Fragments retaining the ability for binding to p53 are indicated with solid lines, and those without the binding ability are indicated with dotted lines.
   (B) His-tagged mortalin deletion mutants were added to COS7 cell lysates and were detected by Western blotting with anti-His tag antibody (input (10%), left panel). p53 immunocomplexes (p53 IC) were analyzed for co-precipitation with mortalin by Western blotting with anti-His tag antibody (right panel). The bands corresponding to the deletion mutants that did not show binding are boxed. Immunoprecipitated p53 was detected by probing the same membrane with anti-p53 (monoclonal) antibody.
   (C) The increase of the amounts of the peptide (comp. pep) corresponding to the mortalin amino acid residues 253-282 to be added reduced the amount of the peptide corresponding to the mortalin amino acid residues 1-435 (mot 1-435) and the peptide corresponding to mortalin amino acid residues 105-435 (mot 105-435). p53 was immunoprecipitated with the polyclonal anti-p53 antibody (CM-1) and detected by Western blotting with a monoclonal anti-p53 antibody (PAb 421))(bands represented with an arrowhead). Co-immunoprecipitated mortalin was detected by Western blotting with anti-His tag antibody (indicated by arrows).
   (D) COS7 cells were transfected with expression constructs encoding V5-tagged mortalin deletion mutant proteins (residues from amino acid residue 250 to 435, and 1 to 435), and the cell lysates were immunoprecipitated with anti-p53 antibody (CM-1, Novocastra). p53 immunocomplexes (lanes 3-4) and input protein (lanes 1-2, 10% of the amount of cell lysate used for immunoprecipitation) were Western blotted with anti-mortalin and anti-V5 tag antibodies to detect endogenous mortalin (indicated by arrow) and mortalin fragments, respectively. The p53 immunocomplexes (IC) were also Western blotted with a monoclonal anti-p53 antibody (Pab 421) (indicated by arrowhead; lanes 3-4, overlapped with V5-tagged mortalin amino acid residues 1-435 in lane 4) to detect immunoprecipitated p53.

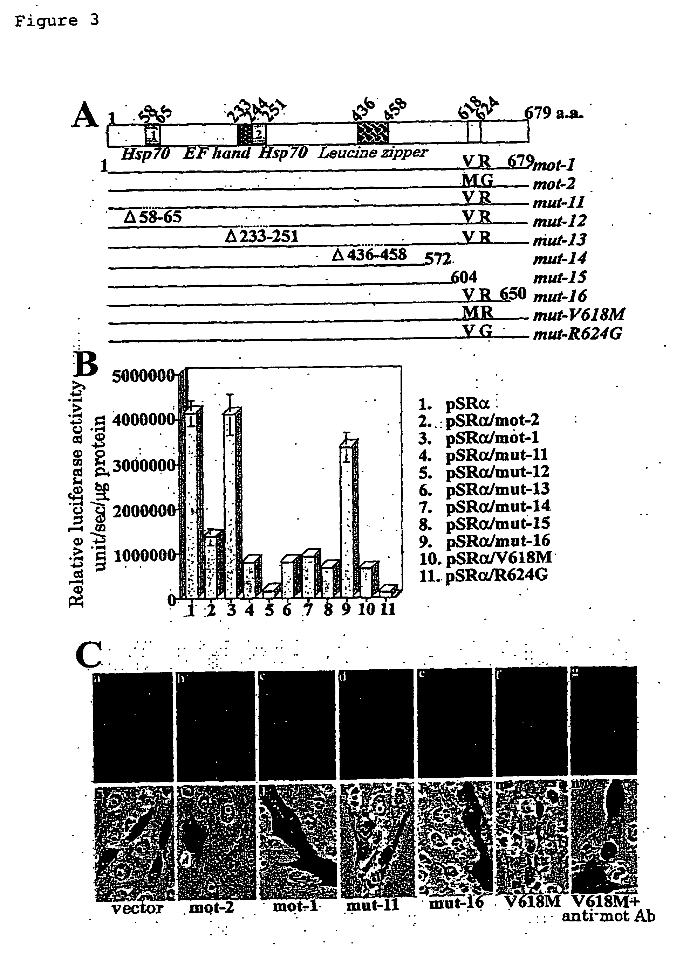

Figure 3 depicts illustration, graph, and photograph (panel C) demonstrating the result of *in vivo* p53-mediated reporter assays in the presence of various mortalin mutants.
   (A) Schematic illustration of the structure of mortalin and mutants thereof. Positions of hsp70, EF hand, and leucine zipper motifs are shown, and deletions of these motifs (mutants 11-13) are shown by dotted lines. The lengths of the three carboxy-terminal deletion mutants (mut-14, -15, and -16) are indicated, and the amino acids at residues 618 and 624 are indicated by single-letter amino acid codes above the line representing each construct.
   (B) Both of the amino acids that are unique to mot-1, together with the three predicted motif regions, were required to prevent mortalin-induced repression of p53-induced transactivation. Co-transfection of the p53 expression plasmid and mortalin constructs that contain one (mut-V618M, mut-R624G) or none (mot-2) of the amino acids of the mot-1-type at amino acids residues 618 and 624 resulted in repression of the p53-responsive reporter activity in p53-/- mouse cells. Removal of any one of the three predicted motifs (mut-11, -10, and -13) or the carboxy-terminal region including amino acid residues 618 and 624 (mut-14 and -15) from mot-1 also resulted in repression of the p53 activity. mot-1 and mut-16 (which retains the amino acids of the mot-1 type at amino acid residues 618 and 624, but lacks the 30 amino acids from the carboxy-terminus) only had a slight effect on the transactivation of p53. pSRα is the control for the blank plasmid vector.
   (C) The p53 activity detected for the p53 responsive β-galactosidase reporter construct was inhibited when a plasmid encoding mot-2 (b and i), mut-11 (d and K), or mutant V16M (f and m) was simultaneously microinjected with the p53 expression plasmid. In contrast, mot-1 (c and j) and mut-16 (e and l) did not decrease the p53-induced transactivation of the reporter. Microinjected cells were visualized by staining with FITC-labeled anti-rabbit IgG (a-g).

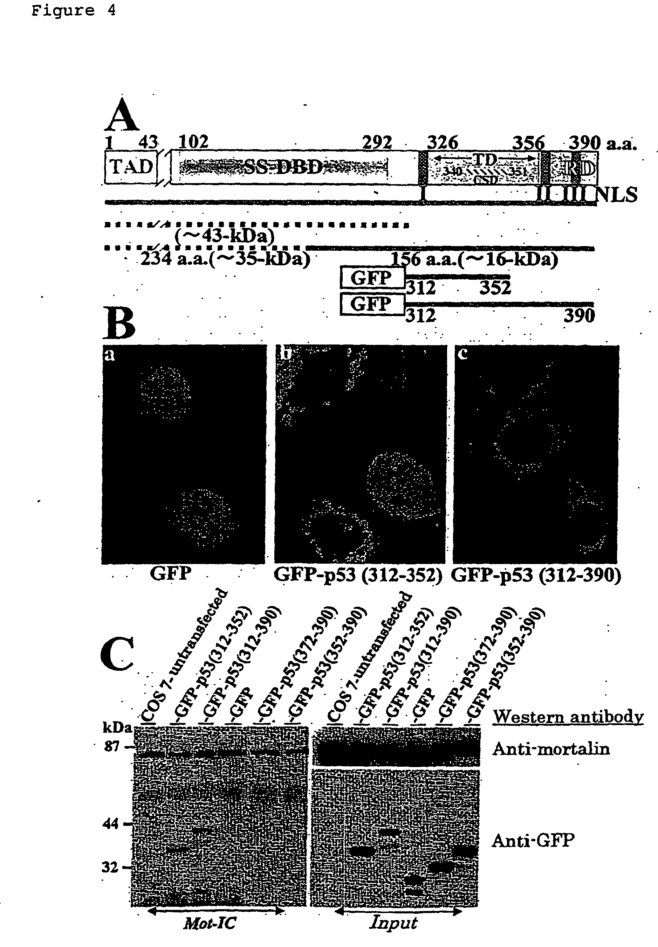

Figure 4 depicts an illustration and photographs (panels (B) and (C)) demonstrating the mortalin binding domain of p53.
   (A) Schematic illustration of the structure of p53 and a p53 deletion mutants. TAD: transactivation domain; SS-DBD: sequence-specific DNA binding domain; NLS I, II, III: nuclear localization signals I, II, and III of amino acid residues 316-325, 369-375, and 379-384, respectively; TD: tetramerization domain; and RD: regulatory domain. The hatched bar indicates the cytoplasmic sequestration domain (CSD). GFP: green fluorescent protein fused with p53. The region that bound with mortalin is indicated with solid lines.
   (B) A photograph showing the green fluorescence of GFP in COS7 cells transfected with (a) GFP alone, (b) GFP-p53 (amino acid residues 312-352), and (c) GFP-p53 (amino acid residues 312-319).
   (C) Mortalin was immunoprecipitated from cells transfected with GFP-tagged p53 fragments with polyclonal anti-mortalin antibodies, and was visualized with monoclonal antibodies. Immunocomplexes (Mot-IC) were also probed with monoclonal anti-GFP antibody to determine whether the p53 fusion proteins co-precipitate with mortalin (left panel). Whereas no precipitation was observed with GFP alone, GFP-p53 (amino acid residues 352-390), and GFP-p53 (amino acid residues 372-390); GFP-p53 (amino acid residues 312-352) and GFP-p53 (amino acid residues 312-390) co-precipitated with mortalin. Bands corresponding to the IgG heavy and light chains are also detected on Mot-IC gel. Input gel (right panel) shows 10% of the sample used for immunoprecipitation.

### Best Mode for Carrying out the Invention

The present invention will be described below in more detail by way of Examples, but the present invention is not limited to these Examples.

### [Example 1] Analysis of interaction between mortalin protein and p53 protein (in vitro binding assay)

After preparing mRNA encoding full-length mouse wild-type p53 with a template of pSP65/p53 plasmid (Braithwaite, A.W. and Jenkins, J.R. (1989) J. Virol. 63, 1792-1799) using Transprobe T Kit (Pharmacia), [³⁵S]-labeled p53 protein was obtained by subjecting to *in vitro* translation reaction using rabbit reticulocyte lysate (Stratagene) for 1 hour in the presence of L- [³⁵S]methionine. After separating the translation products by SDS-PAGE, the translation reaction products were confirmed by autoradiography. The *in vitro* translated p53 protein was purified by immunoprecipitation with anti-p53 antibodies (PAb 421', PAb 1620 (Calbiochem), and PAb 122 (Boehringer-Mannheim)), and was used in the subsequent *in vitro* binding assays described below.

After preparing cDNAs encoding the recombinant mortalin protein (full-length) and various deletion mutants (described later) by PCR and converting them to a form to encode a His-tagged protein by inserting the cDNAs into pQE30 vector (Qiagen), the cDNAs were introduced into *E. coli* for expression. The resulting bacterial extracts were purified with Ni-NTA agarose (Qiagen). The resulting His-tagged recombinant mortalin proteins (0.5 to 1 µg) were mixed with the previously prepared *in vitro* translated [³⁵S]-labeled p53 protein in presence of either in 1 to 2 mg of bovine serum albumin or COS7 cell lysate in Nonidet P-40 solution (400 µl), followed by the addition of anti-mortalin antibody (anti-mtHSP70, Affinity Bioreagents) or anti-p53 antibody (CM1, Novo Castra); and were allowed to incubate for 1 to 2 hours at 4°C. The formed immunocomplexes were mixed for 30 minutes with 20 µl Protein-A/G Sepharose (Gibco) and recovered by centrifugal separation. Moreover, proteins eluted by heating the recovered Sepharose beads to 95°C in a sample buffer containing SDS was separated by SDS-PAGE and transferred to nitrocellulose membrane. Then, p53 protein was detected by autoradiography; mortalin and deletion mutants thereof were detected by Western blotting with anti-His antibody and ECL-chemiluminescence (Amersham).

As a result, as shown in Figure 1B, both the mortalin molecular species of mot-1 and mot-2 specifically bound with full-length p53 protein, while the p53 protein of 43 kDa that lacked the C-terminus portion resulting from the incomplete in vitro translation reaction was verified not to bind with either of the mortalin molecular species.

### [Example 2] Search for p53 binding region using partially deleted mortalin mutant proteins (in vitro binding assay)

To prepare the various partially deleted mortalin mutant proteins shown in Figure 2A, corresponding cDNA fragments were prepared by PCR followed by the preparation of His-tagged proteins according to the method described in Example 1. The resulting partially deleted mortalin mutant proteins were subjected to immunoprecipitation using anti-p53 antibody according to the method described in Example 1, and the co-precipitated proteins were detected by Western blotting using anti-His antibody.

As a result, as shown in Figure 2B, although the fragments of amino acid residues 250-410, 105-282, 105-435, 105-538, and 1-435 retained the ability to bind to p53, the fragments of amino acid residues 105-252, 47-252, and 403-679 clearly lost the binding ability. These results are shown in Figure 2A.

Thus, the region of the mortalin molecule involved in the binding with p53 was considered to be present within the region of amino acid residues 253-282.

Next, a competitive inhibition assay was performed to confirm the involvement of this peptide of amino acid residues 253-282 in the binding to p53. As shown in Figure 2C, the peptide fragment of amino acid residues 253-282 inhibited binding between p53 and mortalin (amino acid residues 1-435 or 105-435) in a dose-dependent manner.

However, since a large amount of peptide was required for the inhibition of the binding with this peptide of amino acid residues 253-282, the competitive effect of the peptide was suggested not to be high. Therefore, the present inventor next expressed the V5-tagged mortalin deletion mutants expressing amino acid residues 250-435, 252-679 (not shown), and 1-435 in COS 7 cells. Their presence in p58-immunocomplexes was analyzed by Western blotting with anti-V5 tag antibody (Figure 2D).

As a result, each of the mortalin deletion mutants was detected in the p53 immunocomplexes (as a single band for the fragment of amino acid residues 250-435, and as multiple bands (degraded products) for the fragment of amino acid residues 1-435).

Notably, only negligible amounts of endogenous mortalin co-precipitated with p53 from cell lysates expressing mortalin fragment of amino acid residues 250-435 (Figure 2D, lane 3) demonstrating that this region can compete efficiently with full-length mortalin for the binding to p53. On the other hand, the peptides of amino acid residues 252-679 and 1-435, which contain a p53 binding region similar to mortalin amino acid residues 250-435, did not compete with full-length mortalin in the binding to p53.

### [Example 3] Search for p53 binding region using partially deleted mortalin mutant proteins (a search based on the inhibition of p53-dependent transcriptional activation)

After preparing cDNAs that encode mutant mortalin proteins missing each of the functions shown in Figure 3A and mortalin proteins point mutated at two different amino acid residues between mot-1 and mot-2 according to the method described in Example 1, the cDNAs were used for p53-dependent reporter analysis. Specifically, cells obtained by stable transfection of plasmid PG130-luc, wherein the firefly luciferase gene has been linked to p53 responsive promoter (having the promoter sequence "GAACATGTCCCAACATGTTG" and expresses the gene downstream of that sequence as a result of binding with p53 protein), and temperature-sensitive mutant p53 protein expression vector pMSVp53Va1135 into mouse embryonic fibroblasts deficient in functional p53 gene (P53-/-MEF) (see, Wadhwa, R. et al. (1998) J. Biol. Chem. 273, 29586-29591) were transfected with various mortalin mutant protein expression vectors and pRL-CMV, serving as an internal control for standardizing transcription efficiency. After incubation at 32.5°C, which is an allowable temperature for the mutant p53 protein, the luciferase activity was measured.

As a result, as shown in Figure 3B, in contrast to the expression of mot-2 protein, which significantly inhibited the p53-dependent transcriptional activation, mot-1 protein did not have any effect on the ability of p53-dependent transcriptional activation, despite the structural difference of only two amino acid residues. Any of the deletions of each functional domain or the regions that were different between mot-1 and mot-2 conferred the inhibition of p53-dependent transcriptional activation similar to mot-2 protein to the mutant mortalin protein. Mutant V618M or R624G, wherein one of the two amino acid residues that differed between mot-1 and mot-2 was made the same amino acid residue as mot-2, exhibited the inhibition of p53-dependent transcriptional activation similar to mot-2, and the effect was particularly strongly observed in mutant R624G. A definitely stronger ability to inhibit p53-dependent transcriptional activation as compared to mot-2 was also observed in the mutant, mut 12 (Δ233-251), wherein the EF hand and Hsp70 homologous domain continuing from EF hand had been deleted, among all the deletion mutants. The activity of mutant mut16 (Δ651-679), wherein the C terminus of the region containing the two amino acid residues that were different between mot-1 and mot-2 had been deleted, was comparable to the activity of mot-1.

Similar results were obtained according to an experiment wherein the above mutant mortalin proteins, temperature-sensitive mutant p53 protein, and p53 responsive β-gal reporter gene pRGCΔfos-lacZ were introduced into a human osteosarcoma cell line Saos2 by microinjection. Namely, as observed in Figure 3C, the p53-dependent transcriptional activation was significantly inhibited by the transfection of an expression vector encoding mot-2, or mutant mot-1 proteins, mut11 or V618M. In contrast, mot-1 or mut16 protein did not have a significant effect on the p53-dependent transcriptional activity similar to the transfection of mouse embryonic fibroblasts (Table 1). In addition, suppression of the p53-dependent transcriptional activation by mot-1 protein having the mutation R624G was significantly inhibited by intracellular injection of anti-mortalin neutralizing antibody (Table 1).

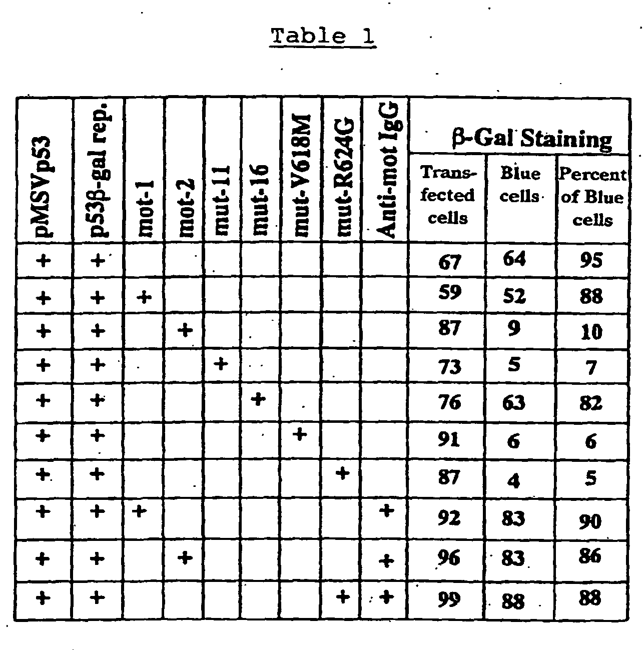

From the above results, the mot-2 protein was suggested to prevent translocation of p53 to the nucleus through the interaction with p53 protein via the region of amino acid residues 253-282, thus inhibiting the transcriptional activation by p53. The substitution of two amino acids in mot-1 was suggested to inhibit the interaction with p53 protein by changes in the secondary structure of the protein or interaction with a different protein (mot-1-p53 binding inhibitory factor).

### [Example 4] Search for regions in p53 protein involved in binding with mortalin

Since the *in vitro* translated product of a 43 kDa truncated p53, lacking the C terminus, lacks the ability to bind to mortalin protein as described in Example 1, it was presumed that this deleted region contains the region that is related to the binding to mortalin. Therefore, as shown in Figure 4A, smaller fragments of this region were prepared and expressed in COS7 cells as GFP-fused protein.

As a result, as shown in Figure 4B, about 90% of the expressed proteins localized to the vicinity of the nucleus in cells transfected with GFP-p53 (amino acid residues 312-352), whereas all proteins localized to the cytoplasm in cells transfected with GFP-p53 (amino acid residues 312-390.

In addition, as a result of immunoprecipitation using anti-mortalin antibody, both GFP-p53 (amino acid residues 312-352) and GFP-p53 (amino acid residues 312-390) were confirmed to bind to mortalin (Figure 4C).

The presence of a tetrarnerization domain and activity regulatory domain have been reported to exist in the C terminus of p53, and the presence of 3 nuclear localization signals (meshed regions I, II, and III in Figure 4A) and one signal related to cytoplasm localization (CSD: amino acid residues 340-351) has been also known to exist in this region. The above results suggest that the binding of mortalin and p53 is mediated by a cytoplasm localization signal sequence of p53, and strongly suggests that binding of mortalin and p53 is involved in the cytoplasmic sequestration previously reported in breast cancer, neuroblastoma, and colon cancer (Ostermeyer, A.G. et al., Proc. Natl. Acad. Sci. USA, 93, 15190-15194, 1996; Stommel, J.M. et al., EMBO J., 18, 1660-1672, 1999).

### Industrial Applicability

According to the present invention, methods of screening for cancer therapeutic agents are provided which utilize the interacting region of p53 and mortalin. As a result, as compared with methods for screening those uses the interaction between full-length proteins, specific cancer therapeutic agents can be screened more efficiently and more economically.

## Claims

1. A method of screening for candidate compounds of cancer therapeutic agents comprising the steps of:
(a) contacting a peptide comprising amino acid residues 253 to 282 of an amino acid sequence of human mot-1 protein also known as mthsp 75 with a peptide comprising amino acid residues 312 to 352 of an amino acid sequence of human p53 in the presence of a test sample;
(b) detecting binding between said peptides; and
(c) selecting the compound that weakens the binding between said peptides, in comparison to binding that occurs in the absence of the test sample.

2. A method of screening for candidate compounds of cancer therapeutic agents comprising the steps of:
(a) contacting a test sample with a cell transfected with the following vectors:
i. a vector containing a DNA encoding a peptide comprising amino acid residues 253 to 282 of an amino acid sequence of human mot-1 protein also known mthsp75,
ii. a vector containing a DNA encoding a peptide comprising amino acid residues 312 to 352 of an amino acid sequence of human p53, wherein said peptide activates human p53 responsive promoter; and
iii. a vector containing human p53 responsive promoter and a reporter gene functionally bound downstream thereof;
(b) detecting the activity of said reporter gene within the cell; and
(c) selecting the compound that increases the reporter expression, in comparison to that occurring in the absence of the test sample.

3. A kit for screening candidate compounds of cancer therapeutic agents comprising:
(a) a peptide comprising amino acid residues 253 to 282 of an amino acid sequence of human mot-1 protein also known as mthsp75; and
(b) a peptide comprising amino acid residues 312 to 352 of an amino acid sequence of human p53.

4. A kit for screening candidate compounds of cancer therapeutic agents comprising:
(a) a vector containing a DNA encoding a peptide comprising amino acid residues 253 to 282 of an amino acid sequence of human mot-1 protein also known as mthsp75;
(b) a vector containing a DNA encoding a peptide comprising amino acid residues 312 to 352 of an amino acid sequence of human p53, wherein said peptide activates human p53 responsive promoter; and
(c) a vector containing human p53 responsive promoter and a reporter gene functionally bound downstream thereof.

## Patentansprüche

1. Verfahren zum Screenen für Kandidatenverbindungen als Krebsheilmittel, umfassend die Schritte:
(a) Inkontaktbringen eines Peptids umfassend die Aminosäurereste 253 bis 282 einer Aminosäuresequenz des menschlichen mot-1-Proteins, auch bekannt als mt hsp75, mit einem Peptid umfassend die Aminosäurereste 312 bis 352 einer Aminosäuresequenz des menschlichen p53 in Gegenwart einer Testprobe;
(b) Nachweisen der Bindung zwischen den Peptiden; und
(c) Auswählen der Verbindung, welche die Bindung zwischen den Peptiden im Vergleich zur Bindung, die in Abwesenheit der Testprobe auftritt, schwächt.

2. Verfahren zum Screenen für Kandidatenverbindungen als Krebsheilmittel, umfassend die Schritte:
(a) Inkontaktbringen einer Testprobe mit einer Zelle, die mit den folgenden Vektoren transfiziert wurde:
i. einem Vektor enthaltend DNA, die ein Peptid umfassend die Aminosäurereste 253 bis 282 einer Aminosäuresequenz des menschlichen mot-1-Proteins, auch bekannt als mt hsp75, codiert;
ii. einem Vektor enthaltend DNA, die ein Peptid umfassend die Aminosäurereste 312 bis 352 einer Aminosäuresequenz des menschlichen p53 codiert, wobei das Peptid den auf menschliches p53 ansprechenden Promotor aktiviert; und
iii. einem Vektor enthaltend den auf menschliches p53 ansprechenden Promotor und ein Reportergen, das stromabwärts davon funktionell gebunden ist;
(b) Nachweisen der Aktivität des Reportergens in der Zelle; und
(c) Auswählen der Verbindung, welche die Reporterexpression im Vergleich zu derjenigen, die in Abwesenheit der Testprobe auftritt, verstärkt.

3. Kit zum Screenen für Kandidatenverbindungen als Krebsheilmittel, umfassend:
(a) ein Peptid umfassend die Aminosäurereste 253 bis 282 einer Aminosäuresequenz des menschlichen mot-1-Proteins, auch bekannt als mt hsp75; und
(b) ein Peptid umfassend die Aminosäurereste 312 bis 352 einer Aminosäuresequenz des menschlichen p53.

4. Kit zum Screenen für Kandidatenverbindungen als Krebsheilmittel umfassend:
(a) einen Vektor enthaltend DNA, die ein Peptid umfassend die Aminosäurereste 253 bis 282 einer Aminosäuresequenz des menschlichen mot-1-Proteins, auch bekannt als mt hsp75, codiert;
(b) einen Vektor enthaltend DNA, die ein Peptid umfassend die Aminosäurereste 312 bis 352 einer Aminosäuresequenz des menschlichen p53 codiert, wobei das Peptid den menschlichen p53-Promotor aktiviert; und
(c) einen Vektor enthaltend den auf menschliches p53 ansprechenden Promotor und ein Reportergen, das stromabwärts davon funktionell gebunden ist.

## Revendications

1. Procédé pour cribler des composés candidats d'agents thérapeutiques anticancéreux comprenant les étapes :
(a) de mise en contact d'un peptide comprenant les résidus d'acides aminés 253 à 282 d'une séquence d'acides aminés de la protéine mot-1 humaine, également appelée mthsp75, avec un peptide comprenant les résidus d'acides aminés 312 à 352 d'une séquence d'acides aminés de la p53 humaine en présence d'un échantillon test ;
(b) de détection d'une liaison entre lesdits peptides ; et
(c) de sélection du composé qui affaiblit la liaison entre lesdits peptides, en comparaison à la liaison qui se produit en l'absence de l'échantillon test.

2. Procédé pour cribler des composés candidats d'agents thérapeutiques anticancéreux comprenant les étapes :
(a) de mise en contact d'un échantillon test avec une cellule transfectée avec les vecteurs suivants :
i. un vecteur contenant un ADN codant un peptide comprenant les résidus d'acides aminés 253 à 282 d'une séquence d'acides aminés de la protéine mot-1 humaine, également appelée mthsp75 ;
ii. un vecteur contenant un ADN codant un peptide comprenant les résidus d'acides aminés 312 à 352 d'une séquence d'acides aminés de la p53 humaine, dans lequel ledit peptide active un promoteur sensible à la p53 humaine ; et
iii. un vecteur contenant un promoteur sensible à la p53 humaine et un gène rapporteur lié de manière fonctionnelle en aval de celui-ci ;
(b) de détection de l'activité dudit gène rapporteur dans la cellule ; et
(c) de sélection du composé qui augmente l'expression du rapporteur, en comparaison à celle qui se produit en l'absence de l'échantillon test.

3. Kit pour cribler des composés candidats d'agents thérapeutiques anticancéreux comprenant :
(a) un peptide comprenant les résidus d'acides aminés 253 à 282 d'une séquence d'acides aminés de la protéine mot-1 humaine, également appelée mthsp75 ; et
(b) un peptide comprenant les résidus d'acides aminés 312 à 352 d'une séquence d'acides aminés de la p53 humaine.

4. Kit pour cribler des composés candidats d'agents thérapeutiques anticancéreux comprenant :
(a) un vecteur contenant un ADN codant un peptide comprenant les résidus d'acides aminés 253 à 282 d'une séquence d'acides aminés de la protéine mot-1 humaine, également appelée mthsp75 ;
(b) un vecteur contenant un ADN codant un peptide comprenant les résidus d'acides aminés 312 à 352 d'une séquence d'acides aminés de la p53 humaine, dans lequel ledit peptide active un promoteur sensible à la p53 humaine ; et
(c) un vecteur contenant un promoteur sensible à la p53 humaine et un gène rapporteur lié de manière fonctionnelle en aval de celui-ci.
